# EUROPEAN PATENT APPLICATION

(11) **EP 1 293 497 A1**
(43) Date of publication of application: **19.03.2003**
(21) Application number: 01943812.6
(22) Date of filing: 22.06.2001
(51) Int. Cl.: C07C 231/24, C07C 235/12, A61K 31/197

(54) **PROCESS FOR THE PRODUCTION OF CALCIUM PANTOTHENATE**

(30) Priority: 23.06.2000 JP 2000188972
(71) Applicant: Daiichi Fine Chemical Co., Ltd., Takaoka-shi, Toyama 933-8511 (JP)
(72) Inventor: TAKAHASHI, Norikazu, Takaoka-shi Toyama 933-8511 (JP); NOZAKI, Tomoko, Takaoka-shi Toyama 933-8511 (JP); EIMORI, Sotohiko, Takaoka-shi Toyama 933-8511 (JP); SHIBA, Motoo, Takaoka-shi Toyama 933-8511 (JP)
(74) Representative: Sternagel, Fleischer, Godemeyer & Partner Patentanwälte
(86) International application number: JP0105354
(87) International publication number: WO01098255

(57) **Abstract**

A method for preparing crystalline calcium pantothenate, which comprises the step of allowing a homogenous mixture containing crystalline calcium pantothenate and amorphous calcium pantothenate to absorb moisture. The aforementioned method comprises, for example, the following steps of (1) mixing crystalline calcium pantothenate and amorphous calcium pantothenate to prepare a homogenous mixture; and (2) allowing the homogenous mixture obtained in the above step (1) to absorb moisture, and enables efficient preparation of crystalline calcium pantothenate with extremely low hygroscopicity.

## Description

### Technical Field

The present invention relates to a method for preparing nonhygroscopic, crystalline calcium pantothenate.

### Background Art

Calcium pantothenate (monocalcium bis[(R)-N-(2,4-dihydroxy-3,3-dimethylbutyryl)-β-alaninate]; hereinafter occasionally abbreviated as "PC") is a medicament listed in the Japanese Pharmacopoeia and widely used for prophylactic and therapeutic treatment of pantothenic acid deficiency as well as therapeutic treatment of contact dermatitis, acute and chronic eczema and the like. This substance is highly dissolvable in water, and when the substance is heated in the state of an aqueous solution, a purity of the substance will be reduced by hydrolysis. Accordingly, a method of spraying an aqueous solution and drying the resulting particles with hot air to produce a product in an amorphous form, or a method of precipitating crystals from a methanol solution, and collecting the precipitates by filtration and then drying with warm air to obtain an amorphous product have been used as methods for a large-scale manufacture of a product with a high purity. However, there is a problem that the amorphous products are hygroscopic and they will receive deliquescence with absorption of moisture at use to give solidified powders.

As crystals of calcium pantothenate, α-form, β-form, and γ-form crystals have been known so far, as well as 4CH₃OH·1H₂O solvate and monohydrate as crystals added with a solvent (see, Inagaki et al., Chem. Pharm. Bull., 24, pp.3097-3102, 1976 as for identification and details of each of the crystal forms). Among them, the 4CH₃OH·1H₂O solvate is hygroscopic and deliquescent. Therefore, it has been desired to develop an industrially applicable method for manufacture of the α-form, β-form, or γ-form crystal, or the monohydrate each of which is nonhygroscopic. However, a method which can stably produce any of these nonhygroscopic crystals in large quantities and in a convenient manner has not yet been developed.

As for preparation of calcium pantothenate, methods having been proposed include a method comprising the steps of crystallizing PC from an organic solvent such as methanol, dissolving the resulting crystals in water, concentrating the resulting solution, and then adding methanol and heating the mixture to obtain nonhygroscopic needles (m.p. 195-196°C) (Levy, H. et al., J. Amer. Chem. Soc., 63, pp.2846-2847, 1941); a method for obtaining crystals from a methanol solution which are different from those obtained by the aforementioned method of Levy et al. (m.p. 153.5-154°C) (Funabashi et al., Bull. Inst. Phys. Chem. Research (Japan), 22, 681, 1943); a method comprising the step of adding an appropriate amount of water to a methanol solution to deposit crystals (Japanese Patent Publication (KOKOKU) No. Sho. 40-2330/1965); a method of depositing an optically active product from a water-containing methanol solution (Japanese Patent Publication (KOKOKU) No. Sho. 49-27168/1974); a method of collecting PC from a methanol solution (Japanese Patent Unexamined Publication (KOKAI) No. Sho. 53-108921/1978); a method of preparing a composition using magnesium lactate and the like (EP394022A1); a method comprising the step of collecting PC from a fermented solution wherein methanol is added to an aqueous solution of PC in a high concentration of about 50 W/V% to adjust a methanol concentration to 90 V/V% (EP822989A1); and a method for preparing PC by using a transformant wherein methanol is added to a PC solution in a high concentration of about 45 to 55 W/W% (EP859848A1).

All of the aforementioned methods involve the use of a mixed solvent of water and an alcoholic solvent such as methanol for crystallization, and are not methods to prepare crystalline PC from a PC solution solely in water. The conventional methods involving the use of methanol for crystallization of PC have a problem of residual methanol, and therefore, it has been desired to develop a method of crystallizing PC from an aqueous solution. However, few attempts have so far been made, since PC is highly dissolvable in water.

### Disclosure of the Invention

An object of the present invention is to provide a method for efficiently preparing nonhygroscopic crystalline calcium pantothenate, and also to provide nonhygroscopic crystalline calcium pantothenate which is substantially free from an organic solvent.

The present inventors made intensive studies to achieve the foregoing object. As a result, it has been found that crystalline calcium pantothenate not suffering from deliquescence due to moisture absorption can be efficiently produced in an industrial scale by vigorously stirring a supersaturated aqueous solution of calcium pantothenate containing calcium pantothenate in a ratio of 60% (W/W) or more, and then drying the resulting viscous crystal suspension, for which they filed a patent application (PCT/JP99/7215). The present inventors made further studies and found that crystalline calcium pantothenate with extremely low hygroscopic property was efficiently producible by allowing a mixture containing crystalline calcium pantothenate and amorphous calcium pantothenate to absorb moisture. The present invention was achieved on the basis of these findings.

The present invention thus provides a method for preparing crystalline calcium pantothenate which comprises the step of allowing a homogenous mixture containing crystalline calcium pantothenate and amorphous calcium pantothenate to absorb moisture. The present invention also provides nonhygroscopic crystalline calcium pantothenate obtainable by the aforementioned method.

The method of the present invention comprises the following steps of:
(1) mixing crystalline calcium pantothenate and amorphous calcium pantothenate to prepare a homogenous mixture; and
(2) allowing the homogenous mixture obtained in the above step (1) to absorb moisture. In the aforementioned moisture absorption step, the homogenous mixture may be left standing, or the mixture may optionally be stirred. Further, the aforementioned moisture absorption step is carried out under humidity and temperature sufficient for crystallization of the amorphous calcium pantothenate into crystalline calcium pantothenate.

The present invention also provides a method for crystallizing amorphous calcium pantothenate into crystalline calcium pantothenate, which comprises the step of allowing the amorphous calcium pantothenate to absorb moisture in the state that the amorphous calcium pantothenate is in contact with crystalline calcium. pantothenate. The present invention also provides nonhygroscopic crystalline calcium pantothenate obtainable by the aforementioned method. The contacting state of the amorphous calcium pantothenate and the crystalline calcium pantothenate can generally be attained by preparing a homogenous mixture of the both. By allowing the resulting homogenous mixture of amorphous calcium pantothenate and crystalline calcium pantothenate to absorb moisture under humidity and temperature sufficient for crystallization of the amorphous calcium pantothenate into crystalline calcium pantothenate, the amorphous calcium pantothenate absorbs a required amount of moisture, and thus crystallization of the whole homogenous mixture advances.

The present invention further provides a homogenous mixture obtained by mixing crystalline calcium pantothenate and amorphous calcium pantothenate, and use of the homogenous mixture for the preparation of crystalline calcium pantothenate. By allowing this homogenous mixture to absorb moisture under humidity and temperature sufficient for crystallization of the amorphous calcium pantothenate into crystalline calcium pantothenate, crystalline calcium pantothenate can be prepared.

### Brief Description of Drawings

Fig. 1 is a graph showing advance of crystallization into α-form crystals with time observed in Example 1.
Fig. 2 is a graph showing advance of crystallization into β -form crystals and change in moisture content with time observed in Example 7.
Fig. 3 includes graphs showing the relationship between temperature or humidity and advance of crystallization. In the figure, Fig. 3(A) shows the results for a homogenous mixture obtained by mixing equal amounts of α-form PC and amorphous PC, and Fig. 3 (B) shows the results for a homogenous mixture obtained by mixing equal amounts of β-form PC and amorphous PC.
Fig. 4 is a graph showing the results of moisture absorption test for the calcium pantothenates each consisting of α-form crystals and the β-form crystals obtained in Examples 1 or 7. The results for amorphous calcium pantothenate as a control are also shown.

### Best Mode for Carrying Out the Invention

The entire disclosure of Japanese Patent Application No. 2000-188972 (filed on June 23, 2000) is incorporated in the disclosure of the present specification by reference.

The term "crystalline" used in the specification encompasses a substance containing a small amount of an amorphous part, as well as a substance substantially consisting completely of crystals. However, calcium pantothenate in a completely amorphous state (the state which gives substantially no detectable peak by the powder X-ray diffraction analysis) is excluded. The term "crystalline" should not be interpreted to exclude a substance containing a small amount of an amorphous part. Further, the term "amorphous" means a state in which no peak is substantially detectable by powder X-ray diffraction analysis. The term "nonhygroscopic" used in the specification means that a moisture absorption amount is 2% or less, preferably 1% or less, after 24 hours at 40°C under a relative humidity of 75%.

The calcium pantothenate provided by the method of the present invention is a nonhygroscopic crystalline substance comprising nonhygroscopic crystals of calcium pantothenate. The calcium pantothenate prepared by the method of the present invention comprises at least one kind of nonhygroscopic crystals of calcium pantothenate, which exists as a crystalline substance as a whole and is nonhygroscopic as a whole. Although the calcium pantothenate provided by the method of the present invention may contain an amorphous part, it essentially contains a nonhygroscopic crystalline component in an amount sufficient for satisfying the requirement of nonhygroscopic property defined above. The calcium pantothenate provided by the method of the present invention is preferably provided as a crystalline substance not substantially containing any amorphous part. Whether calcium pantothenate prepared by the method of the present invention is nonhygroscopic can be readily verified by an ordinary moisture absorption test.

The calcium pantothenate prepared by the method of the present invention may contain as a nonhygroscopic crystal α-form crystal, β-form crystal, γ-form crystal, or monohydrate crystal, or a mixture thereof. When the calcium pantothenate prepared by the method of the present invention is obtained as mixture of the aforementioned nonhygroscopic crystals, it may contain any combination thereof. The crystals may be present at any content ratios. Characteristic features and classification of the aforementioned crystals are described in detail in the article by Inagaki et al. (Chem. Pharm. Bull., 24, pp.3097-3102, 1976). In general, each of these crystals is definitely distinguishable by powder X-ray diffraction and infrared absorption spectrum, and therefore each of the crystals can be identified. The aforementioned four forms of the crystals have been known so far as nonhygroscopic crystals; however, there is a possibility that a nonhygroscopic crystal other than these four forms of the crystals may exist. The crystalline calcium pantothenate prepared by the method of the present invention may contain such a novel nonhygroscopic crystal. The crystals of calcium pantothenate may sometimes cause a transition, and a crystalline form may sometimes change depending on conditions for the moisture absorption step or during a preparation step such as stirring.

The method of the present invention relates to preparation of nonhygroscopic crystalline calcium pantothenate, which is characterized to comprise the step of allowing a homogenous mixture containing crystalline calcium pantothenate and amorphous calcium pantothenate to absorb moisture. The method of the present invention generally comprises the following steps of:
(1) mixing crystalline calcium pantothenate and amorphous calcium pantothenate to prepare a homogenous mixture; and
(2) allowing the homogenous mixture obtained in the above step (1) to absorb moisture.

The first step of the method of the present invention is a step of mixing crystalline calcium pantothenate and amorphous calcium pantothenate to prepare a homogenous mixture comprising crystalline calcium pantothenate and amorphous calcium pantothenate. As the crystalline calcium pantothenate, nonhygroscopic crystalline calcium pantothenate is preferably used. For example, nonhygroscopic α -form crystal, β-form crystal, γ-form crystal, or monohydrate crystal, or a mixture thereof may be used. Among them, α-form crystal, β-form crystal or a mixture thereof is preferably used. The method for preparing amorphous calcium pantothenate used as a starting material for the method of the present invention is not particularly limited. For example, preferably used amorphouses include those obtained by a method comprising the steps of spraying an aqueous solution of calcium pantothenate and drying the sprayed solution with hot air to prepare amorphous powder or a method comprising the steps of crystallizing calcium pantothenate from a methanol solution, collecting the crystals by filtration and drying the crystals with hot air to prepare amorphous powder of calcium pantothenate.

The calcium pantothenate used as a starting material desirably has a purification degree as high as possible. For example, calcium pantothenate prepared by a synthetic method, a method comprising fermentation, or a method applied by a gene recombination technique and the like may be used. The calcium pantothenate may be purified by recrystallization or a conventional purification means to prepare crystalline calcium pantothenate or amorphous calcium pantothenate.

The method for mixing crystalline calcium pantothenate and amorphous calcium pantothenate is not particularly limited. It is usually desirable to prepare a homogenous mixture by mechanically mixing crystalline calcium pantothenate and amorphous calcium pantothenate prepared as powder. Particle size of powdery crystalline calcium pantothenate or amorphous calcium pantothenate used as a raw material is not particularly limited. For example, the particle size is about 20 to 500 µm. The preparation of homogenous mixture can be carried out by a method widely used in the field or art as means for mixing solid, preferably powder.

A temperature and humidity at the preparation of the homogenous mixture are not particularly limited. For example, the mixing may be carried out at room temperature under ordinary humidity, for example, 40 to 80% RH (% RH represents relative humidity, the same shall apply hereinafter). It is also possible to prepare the homogenous mixture under appropriate warming and humidification conditions, thereby the second step can be performed simultaneously. Mixing ratio of crystalline calcium pantothenate and amorphous calcium pantothenate is not particularly limited, and the ratio can be suitably chosen by those skilled in the art depending on the moisture absorption conditions in the subsequent step, desired form of crystalline calcium pantothenate and the like. In general, 10% by weight or more, preferably about 30% by weight, of crystalline calcium pantothenate may be used based on the total weight of the homogenous mixture.

The second step of the method of the present invention is a step of allowing the homogenous mixture of crystalline calcium pantothenate and amorphous calcium pantothenate obtained in the above step to absorb moisture. In general, this moisture absorption step can be carried out by leaving the aforementioned homogenous mixture standing under appropriate temperature and humidity, or stirring the homogenous mixture under appropriate temperature and humidity. The step is carried out under temperature and humidity sufficient for crystallization of amorphous calcium pantothenate into nonhygroscopic crystalline calcium pantothenate. Such temperature and humidity can be readily determined by those skilled in the art depending on, for example, type of the homogenous mixture, form of the desired nonhygroscopic calcium pantothenate and the like by performing a test similar to that described in Example 8 of the specification. For example, a suitable combination of a temperature of from room temperature to about 80°C and humidity of about 30 to 90% RH, preferably about 40 to 80% RH, can be chosen.

Means for performing the mixing used for the moisture absorption is not particularly limited, and an ordinary mechanical stirring apparatus can be used. In order to efficiently prepare the target product in an industrial scale, stirring is generally indispensable. The term "stirring" used in the present specification should be interpreted as its broadest meaning including common stirring operations, as well as means capable of achieving physical effect similar to the stirring (e.g., vibrations, fluidization, ultrasonic stirring and the like).

The nonhygroscopic crystalline calcium pantothenate obtained in the second step preferably substantially consists of nonhygroscopic crystalline calcium pantothenate, and preferably prepared as a crystalline substance that does not substantially contain an amorphous part. Nonhygroscopic crystalline calcium pantothenate may sometimes be obtained which contains a form of crystalline calcium pantothenate different from that of the crystalline calcium pantothenate used for the preparation of the homogenous mixture.

In the aforementioned second step, the desired nonhygroscopic calcium pantothenate can be obtained without any treatment such as drying. An optional treatment such as drying and granulation may be applied as required. Means for the drying is not particularly limited, and the drying may be performed by using a drier available in this field under appropriate conditions.

### Examples

The present invention will be explained more specifically by referring to examples. However, the scope of the present invention is not limited to the following examples.

### Example 1

Crystalline calcium pantothenate (PC) of α-form crystals obtained from a methanol solution by a known method was used as nonhygroscopic PC. Peak positions in powder X-ray diffraction of the α-form crystalline PC were identical to those of known α-form crystalline PC (Inagaki et al., Chem. Pharm. Bull., 24, pp.3097-3102, 1976). The moisture content of the α-form crystalline PC was 1.5 to 2.5% (Karl Fischer method).

An aqueous solution of the calcium pantothenate was dried in a spray drier to obtain amorphous PC. The moisture content of the resulting amorphous PC was 2 to 3% (Karl Fischer method). Substantially no peak was observed by powder X-ray diffraction measurement.

MultiFlex 2kW (horizontal type goniometer, Rigaku Corporation) was used as an X-ray diffraction apparatus, and the following conditions were applied for the X-ray diffraction measurement.
X-ray tube target: Cu
Tube voltage: 40 kV, Tube current: 20 mA

Monochromatization of X-ray: monochromator method + PHA (pulse height analyzer, differentiation mode)
Slit:
   Diverging slit 1°
   Scattering slit 1°
   Light-receiving slit 0.15 mm
Sampling interval: 0.02°
Scanning velocity: 5° /min

For the measurement, a sample exchanger (ASC-6A) was used, in which rotation number was set to be 60 rpm. As for a sample holder, 0.8 g of sample was filled in a penetration type sample plate, or a sample plate with a bottom (deep bottom type, depth: about 2 mm) was used. The total amount of the sample in a container for standing was transferred to a mortar, ground with a pestle until coarse particles totally disappeared, and filled in each sample holder according to the method described in the operation manual. X-ray intensity count value was calculated as a value of variation of peak area with time in each experimental example by using pattern processor software of MDI (Materials Data Inc.), Jade (Ver. 5), Windows version.

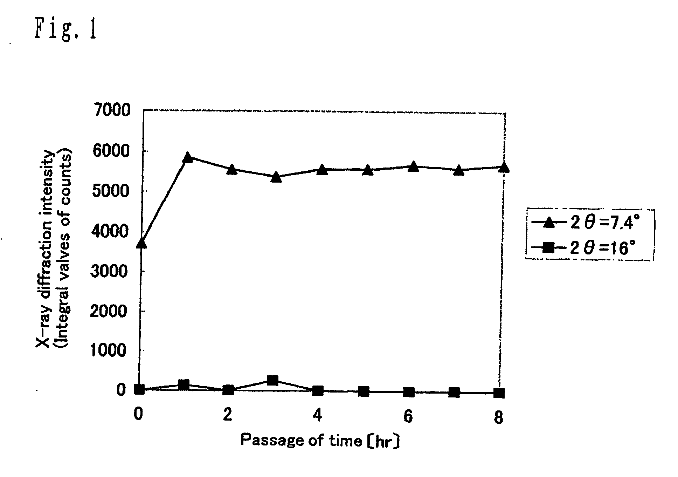

α-Form crystalline PC (75.0 g) and 75.0 g of amorphous PC (ratio of α-form crystals: 50%) were placed in a 1 L-volume eggplant-shaped flask, and after nitrogen substitution, homogenously mixed for 1 hour in a rotary evaporator at 50-60 rpm. The homogenous mixture was weighed and introduced into containers for standing (square-shaped styrol cases, material: antistatic styrol resin, external dimension: 100 x 65 x 28 (mm)) in an amount of 5.0 g for each container, and uniformly spread. The containers containing the crystals were left standing in a thermo/humidistat (LH20-11M, Nagano Science Equipment Mfg.) set at 70°C and 80% RH, and measurements of powder X-ray diffraction and moisture content (Karl Fischer method) were performed every 1 hour. In the X-ray diffraction, 7.4° for α-form crystals and 16° for β-form crystals were used as characteristic diffraction angles to determine changes with time in integral values of their counts and moisture contents. The results showing progress of crystallization are shown in Fig. 1. It was observed that α-form crystals increased with the passage of time. The peak positions in the powder X-ray diffraction chart were identical to those reported by Inagaki et al. (Chem. Pharm. Bull., 24, pp.3097-3102, 1976). It was also observed that the moisture content increased once and then decreased with the passage of time.

### Example 2

In the same manner as in Example 1, a homogenous mixture was prepared with a ratio of α-form crystals of 50%, weighed into containers for standing in an amount of 5.0 g for each container, and uniformly spread. The containers containing the crystals were left standing in a thermo/humidistat set at 40°C and 70% RH, and measurements of powder X-ray diffraction and moisture content (Karl Fischer method) were performed every 1 hour to determine changes with time in integral values of their counts and moisture contents. With the passage of time, α-form crystals increased, and peaks unique to β-form crystals appeared. Thus, generation and increase of β-form crystals were verified. It was also observed that the moisture content first increased and then decreased with the passage of time.

### Example 3

β-Form crystalline PC was obtained from a water-containing ethanol solution of calcium pantothenate by a known method. By powder X-ray diffraction, it was verified that peak positions of the product were identical to those of the β-form crystals reported by Inagaki et al., Chem. Pharm. Bull., 24, pp.3097-3102, 1976. Equal amounts of the resulting β-form crystalline PC and amorphous PC were placed in an eggplant-shaped flask and homogenously mixed in a rotary evaporator. The homogenous mixture obtained was spread in a vat with a thickness of 3 to 5 mm and left standing under conditions of 40°C and 75% RH for 20 hours or more to obtain β-form crystalline PC. Moisture content of the resulting β-form crystals was 1.3% (Karl Fischer method), and the peak positions in the powder X-ray diffraction chart were identical to those reported by of Inagaki et al. It was observed that the moisture content first increased and then decreased with the passage of time.

### Example 4

The β-form crystalline PC (75.0 g) obtained in Example 3 and 75.0 g of amorphous PC were placed in a 1 L-volume eggplant-shaped flask, and after nitrogen substitution, homogenously mixed for 1 hour in a rotary evaporator at 50 to 60 rpm. In the same manner as in Example 1, the resulting homogenous mixture was weighed into containers for standing in an amount of 5.0 g for each container, and uniformly spread. The containers containing the homogenous mixture were left standing in a thermo/humidistat set at 60°C and 50% RH, and measurements of powder X-ray diffraction and moisture content (Karl Fischer method) were performed every 1 hour to determine changes with time in integral values of their counts and moisture contents. Increase of β-form crystals with the passage of time was observed. It was also observed that the moisture content first increased and then decreased with the passage of time.

### Example 5

β-Form crystalline PC (45.0 g) and 105.0 g of amorphous PC (ratio of β -form crystalline PC: 30%) were placed in a 1 L-volume eggplant-shaped flask, and after nitrogen substitution, homogenously mixed for 1 hour in a rotary evaporator at 50 to 60 rpm. In the same manner as in Example 1, the resulting homogenous mixture was weighed into containers for standing in an amount of 5.0 g for each container, and uniformly spread. The containers containing the homogenous mixture were left standing in a thermo/humidistat set at 60°C and 50% RH, and measurements of powder X-ray diffraction and moisture content (Karl Fischer method) were performed every 1 hour to determine changes with time in integral values of their counts and moisture contents. Increase of β -form crystals with the passage of time was observed. It was also observed that the moisture content first increased and then decreased with the passage of time.

### Example 6

β-Form crystalline PC (75.0 g) and 75.0 g of amorphous PC (ratio of β-form crystalline PC: 50%) were placed in a 1 L-volume eggplant-shaped flask, and after nitrogen substitution, homogenously mixed for 1 hour in a rotary evaporator at 50 to 60 rpm. In the same manner as in Example 1, the resulting homogenous mixture was weighed into containers for standing in an amount of 5.0 g for each container, and uniformly spread. The containers containing the crystals were left standing in a thermo/humidistat set at 40°C and 70% RH, and measurements of powder X-ray diffraction and moisture content (Karl Fischer method) were performed every 1 hour to determine changes with time in integral values of their counts and moisture contents. Increase of β-form crystals with the passage of time was observed. It was also observed that the moisture content first increased and then decreased with the passage of time.

### Example 7

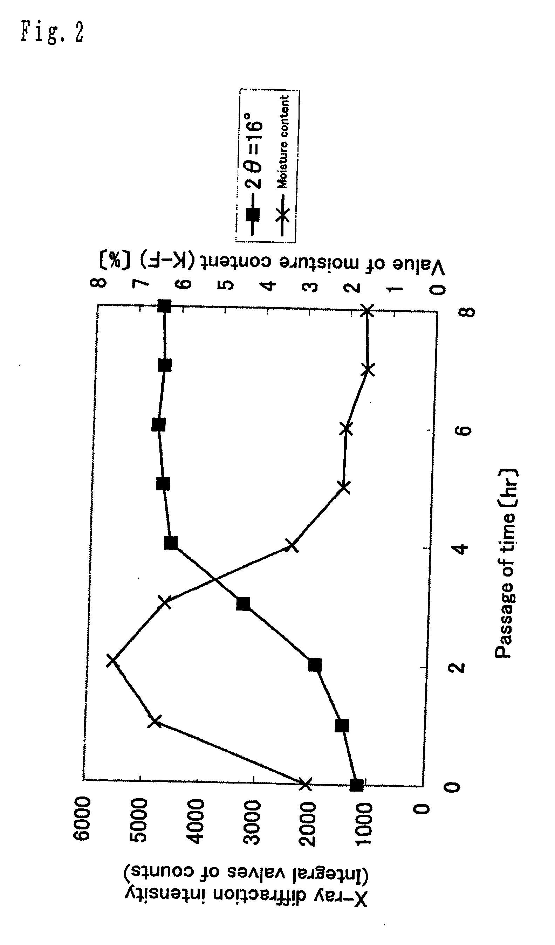

β-Form crystalline PC (45.0 g) and 105.0 g of amorphous PC (ratio of β -form crystalline PC: 30%) were placed in a 1 L-volume eggplant-shaped flask, and after nitrogen substitution, homogenously mixed for 1 hour in a rotary evaporator at 50 to 60 rpm. In the same manner as in Example 1, the resulting homogenous mixture was weighed into containers for standing in an amount of 5.0 g for each container, and uniformly spread. The containers containing the crystals were left standing in a thermo/humidistat set at 40°C and 70% RH, and measurements of powder X-ray diffraction and moisture content (Karl Fischer method) were performed every 1 hour to determine changes with time in integral values of their counts and moisture contents. Increase of β-form crystals with the passage of time was observed. The results are shown in Fig. 2.

### Example 8

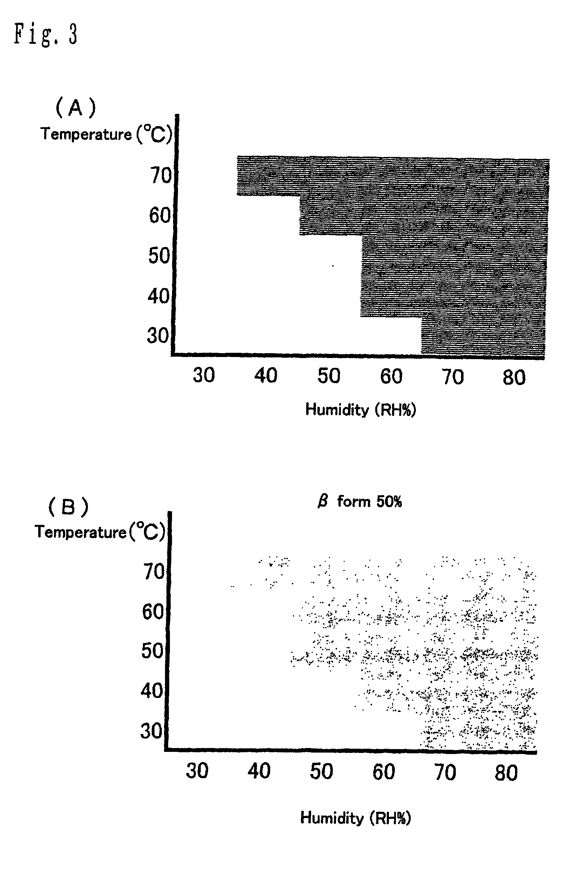

In the same manner as in Example 1, a homogenous mixture was prepared by using equal amounts of α-form crystalline PC and amorphous PC. Further, in the same manner as in Example 3, a homogenous mixture was prepared by using equal amounts of β-form crystalline PC and amorphous PC. In the same manner as in Example 1, each of the resulting homogenous mixtures was left standing for 24 hours under various temperatures and humidities, and measurements of powder X-ray diffraction and moisture content (Karl Fischer method) were performed every 1 hour to determine changes with time in integral values of their counts and moisture contents, thereby temperature and humidity conditions for crystallization of the amorphous PC were investigated. The results are shown in Fig. 3. For each container, it was observed that the moisture content first increased and then decreased with the passage of time.

In the both experiments utilizing α-form crystalline PC and β-form crystalline PC, it was observed that crystallization proceeded in appropriate regions of temperature and humidity. Further, when α-form crystalline PC was used, no growth of β-form crystals was observed in a high humidity region, but only α-form crystals grew quickly. When β-form crystalline PC was used, β-form crystals solely grew in a wide range of conditions, and only a slight growth of α-form crystals was observed in the region of the maximum temperature and the maximum humidity

### Example 9

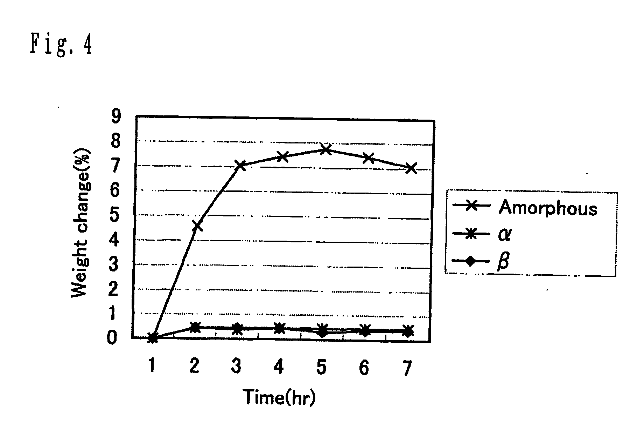

A moisture absorption test was performed under conditions of 40°C and 75% RH for the PC consisting of α-form crystals obtained in Example 1 and the PC consisting of β-form crystals obtained in Example 7. A similar test was performed for the amorphous PC used in Example 1 as a control. The results are shown in Fig. 4. Both of the PC consisting of α-form crystals obtained in Example 1 and the PC consisting of β-form crystals obtained in Example 7 showed weight change of 1% by weight or less after 7 hours and 24 hours, and thus it was verified that they were nonhygroscopic.

### Industrial Applicability

According to the method of the present invention, hygroscopic crystalline calcium pantothenate with extremely low hygroscopicity can be efficiently prepared.

## Claims

1. A method for preparing crystalline calcium pantothenate, which comprises the step of allowing a homogenous mixture containing crystalline calcium pantothenate and amorphous calcium pantothenate to absorb moisture.

2. A method for preparing crystalline calcium pantothenate, which comprises the following steps of:
(1) mixing crystalline calcium pantothenate and amorphous calcium pantothenate to prepare a homogenous mixture; and
(2) allowing the homogenous mixture obtained in the above step (1) to absorb moisture.

3. The method according to claim 1 or claim 2, wherein the moisture absorption is carried out under humidity and temperature sufficient for crystallization of the amorphous calcium pantothenate into crystalline calcium pantothenate.

4. The method according to any one of claims 1 to 3, wherein the crystalline calcium pantothenate contained in the homogenous mixture is α-form crystal, β -form crystal, γ-form crystal, or monohydrate crystal, or a mixture thereof.

5. The method according to any one of claims 1 to 4, wherein the crystalline calcium pantothenate substantially consists of α-form crystal, β-form crystal, γ -form crystal, or monohydrate crystal, or a mixture thereof.

6. A method for crystallizing amorphous calcium pantothenate into crystalline calcium pantothenate, which comprises the step of allowing the amorphous calcium pantothenate to absorb moisture in a state that the amorphous calcium pantothenate is in contact with crystalline calcium pantothenate.

7. The method according to claim 6, wherein the contacting state of the amorphous calcium pantothenate and the crystalline calcium pantothenate is attained by preparing a homogenous mixture of the amorphous calcium pantothenate and the crystalline calcium pantothenate.

8. Nonhygroscopic crystalline calcium pantothenate, which is obtainable by the method according to any one of claims 1 to 7.

9. A homogenous mixture containing crystalline calcium pantothenate and amorphous calcium pantothenate, which is for use in preparation of crystalline calcium pantothenate.
